# EUROPEAN PATENT APPLICATION

(11) **EP 1 293 554 A1**
(43) Date of publication of application: **19.03.2003**
(21) Application number: 01941121.4
(22) Date of filing: 20.06.2001
(51) Int. Cl.: C11B 9/00, A61K 7/46, A61K 35/78, A61P 25/22, A61P 25/20

(54) **STRESS-RELIEVING PERFUMES AND STRESS-RELIEVING PERFUME COMPOSITIONS CONTAINING THE SAME**

(30) Priority: 20.06.2000 JP 2000184132
(71) Applicant: Shiseido Co., Ltd., Tokyo 104-8010 (JP)
(72) Inventor: SHOJI, Ken Shiseido Research Center(Shin-Yokohama), Yokohama-shi, Kanagawa 224-8558 (JP); SAKAI, Keiko, Shiseido Res. Center(Shin-Yokohama), Yokohama-shi, Kanagawa 224-8558 (JP)
(74) Representative: Henkel, Feiler, Hänzel
(86) International application number: JP0105261
(87) International publication number: WO01098442

(57) **Abstract**

The object of the invention is to provide perfumes that is effective on physiologically manifested stresses and the perfume compositions comprising the same that is effective on stress reduction. The invention provides the stress relieving perfumes wherein the active component is valerian oil, especially fatty acid-removed valerian oil, and stress relieving perfume compositions containing the same.

## Description

This application claims the priority of Japanese Patent Application No.2000-184132 filed on June 20, 2000, which is incorporated herein by reference.

### FIELD OF THE INVENTION

This invention relates to a stress relieving perfume and a perfume composition containing the same, and particularly relates to a stress relieving perfume that reduces stresses having harmful influence on physiological function.

### BACKGROUND OF THE INVENTION

Stresses in the modern society sometimes appear in various physiological forms including but not limited to allergies and a gastric ulcer. However, such conditions do not always become so serious as to need a medical treatment. Therefore, the use of medicines which are often associated by supervision of a doctor, such as oral administration of medicine or administration of medicine by injection or the like, are limited. In addition, the use of such medicines bear the risk of producing side effects.

On the other hand, in aromatherapy, an attempt has been made to obtain stress relieving effect by using some kind of natural essential oils where relaxation effects or cooling effects have been confirmed in traditional remedies, and such attempt exhibits its effectiveness in alleviating stress induced phychological conditions. This method has the advantage that it does not add new stresses when the essential oil is given to human body.

However, it has been determined by experiences of experts that which essential oils should be selected to obtain stress relieving effect among many kinds of essential oils. Thus the effects recognized by each person are subjective and indefinite, and are greatly different among individuals. And a stress relieving perfume that shows effect on relieving physiological stresses has been needed.

On the other hand, even if an essential oil could have the stress reducing effect, sometimes it made work efficiency decrease by over-relaxing the treated individual. Thus there exists a need to select the essential oil which can give a stress relieving effect with a moderate strain condition maintained.

In addition, problems regarding blends of perfumes are occur often, because fragrances containing essential oils used for relieving stresses sometimes do not suit people's sensory preferences. This posses new problems in that, depending on the situation, there is a risk that fragrances that do not suit people's sensory preferences cause new stresses. Therefore, it must be avoided using special fragrances less desirable in scent to obtain a stress relieving effect. And it has been needed to find a perfume by which the balance of fragrance is not lost even if they were compounded in large quantities.

### SUMMARY OF THE INVENTION

The object of the invention is to provide a stress relieving perfume wherein the active component is a perfume effective in relieving stresses with physiological manifestations, and perfume compositions using the same.

The inventors have found that valerian oil, especially valerian oil in which the malodorous fatty acids were removed with alkali treatment, have stress relieving effects in physiological aspect.

Namely, the invention is characterized by that the active component is valerian oil.

In addition, in the stress relieving perfumes of the invention, it is preferred that the malodorous fatty acid of the said valerian oil has been removed.

Also, it is preferred that the stress relieving perfume contains more than 0.2 % by weight of valerian oil.

Moreover, the stress relieving perfume compositions of the invention contains the said stress relieving perfume.

Furthermore the method of the invention for relieving stresses and the use as stress relieving agents is characterized by inhalation of an effective quantity of valerian oil.

### BRIEF DESCRIPTION OF THE DRAWINGS

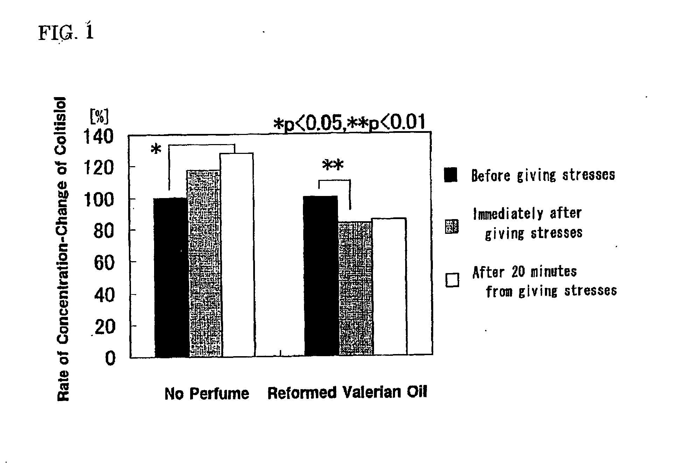

Fig. 1 shows concentration-change of cortisol, a stress indicator, in saliva by using the stress relieving perfume of the invention.

### DETAILED DESCRIPTION OF THE PREFERRED EMBODIMENT

Essential oils obtained from a rhizome of European valerian or its close species such as valerian (*Valerian officinalis* L. var *latifolia* Miq. (*V*. *japonica* Makino)), Yezo valerian (*V. fauriei* form *yezoensis*) and Indian valerian (*V. wallichii* D.C.) or the like by treating them with methods well known in the art such as steam distillation or solvent extraction can be used as valerian oils used by the invention. Also the essential oils extracted from European valerian and its close species are commercially available as valerian oil, valerian fauriei oil and Kisso root oil (Japanese valerian oil), and these can be used regardless of source (*e.g*., Japan, China or Europe).

In general, the above-mentioned natural valerian oil includes at least one kind of fatty acids, usually more than several kinds of fatty acids, especially malodorous acids such as acetic acid and isovaleric acid. It is preferred in the invention that valerian oil where these malodorous acids are removed is used.

Fatty acid-removed valerian oil according to the invention can be obtained by removing the acidic components, including fatty acid, from the above natural valerian oil with an alkali treatment.

The concrete method for manufacturing malodorous fatty acid-removed valerian oil is to dissolve valerian oils containing the malodorous fatty acids in an organic solvent, preferably in ether, then extract and remove the acidic components including fatty acids by conducting an extraction operation with addition of alkali water solution thereto. Alkali water solution of both inorganic base and organic base can be used if it is generally used in an extraction operation, however it is preferred that alkali water solution is sodium bicarbonate solution or sodium hydroxide solution. Especially it is preferred that valerian oil is extracted with sodium bicarbonate solution first, then with sodium hydroxide solution.

The obtained organic solvent stratum is dried with anhydrous magnesium sulfate, then the organic solvent is removed by distillation under reduced pressure to acquire valerian oil in which a fatty acid is removed. This method is disclosed in Japanese patent laid open No. 09-24302.

Also the malodorous components of the said fatty acid can be removed by eliminating a part of a low boiling point lower than 80 degrees with a vacuum distillation as described in Japanese patent laid open No. 1-254628, however, this method has some fear to denature stress relieving components because this method is conducted under heating condition, and also the yield by this method is not so high. Therefore the method by the said alkali treatment is more preferable.

Valerian oil according to the invention, especially valerian oil with the malodorous components removed has excellent stress reducing effects.. The stress reducing effects of the invention is not merely psychological recognition, and the action reducing the concentration of cortisol in the human body, known as a stress indicator, is recognized as the effects. It is known that cortisol is made in an adrenal gland, and that the concentration of cortisol in blood rises by feeling stresses. Moreover, as it is known that the concentration of cortisol in serum and the concentration of cortisol in saliva are correlated, the concentration of cortisol in saliva can be used as a stress indicator by measuring it with the RIA solid phase method. Accordingly, since the stress relieving perfumes of the invention reduce the concentration of cortisol in saliva, it is effective in relieving the physiological manifestations of stress.

It is preferred that the stress relieving perfume of the invention contains more than 0.2 % by weight of valerian oil. If the amount of the valerian oil is less than 0.2 % by weight, the desired stress relieving effects is less probable.

The stress relieving perfumes of the invention can be used not only to obtain stress relieving effects by inhaling valerian oil directly, but also used as a stress reducer in perfume compositions by being compounded in fragrance products. For example, it can be used in perfume, cologne, shampoo and rinse, skin care products, body shampoo, body rinse, body powder, aromatics, deodorants, or bath agents.

Examples of the invention and tests for confirming stress relieving effects of the invention are concretely disclosed below:
The inventors paid attention to the point that valerian oil, especially malodorous fatty acid-removed valerian oil (hereinafter "reformed valerian oil") has a highly effective sedative effect. Then it was attempted to confirm the stress relieving effect of reformed valerian oil by measuring cortisol in saliva.

First, the method for producing reformed valerian oil used in the examples and tests of the invention is shown.

### Example 1: Stress reducing perfume

20g of commercially available valerian oil (Valerian root oil: YAMAMOTO PERFUME INC.) was dissolved with 200 ml of diethyl ether. Extraction was done three times with 100 ml of saturated sodium bicarbonate solution. Moreover, extraction of the obtained ether stratum was done three times with 100 ml of 5 % sodium hydroxide solution. The obtained ether stratum was dried with anhydrous magnesium sulfate, then diethyl ether was removed by evaporation under reduced pressure with a rotary evaporator. Finally 18.3g (yield: 91.5%) of reformed valerian oil (the stress relieving perfume) with the malodorous fatty acids removed was obtained.

### Test Example 1: Fragrance Sensory Test

The perfumes of both the reformed valerian oil made by the method described in Example 1 above and the valerian oil used as a material without alkali treatment were evaluated by 10 professional panelists. The criterion for evaluation is as follows.

### <Criterion for evaluation>

ⓞ more than 9 out of 10 people evaluated there was no unpleasant smells
○ 6 to 8 out of 10 people evaluated there was no unpleasant smells.
Δ 3 to 5 out of 10 people evaluated there was no unpleasant smells.
× less than 2 out of 10 people evaluated there was no unpleasant smell.

**Table 1**

| Sensory evaluation of valerian oil | |
|---|---|
| Reformed valerian oil | ⓞ |
| (Example 1) | |
| Valerian oil | × |
| (without removed fatty acids) | |

As apparent from the above Table 1, valerian oil without removed fatty-acids had an unpleasant odor. This unpleasant odor has a chance of giving people new stresses. To the contrary, the perfume of reformed valerian oil in which acid ingredients such as a fatty acid are removed with alkali treatment has no unpleasant odor, thus this perfume of the invention is more preferable. Therefore, reformed valerian oil in which fatty acids have been removed can be used independently as a stress relieving perfume. And even if a large quantity of reformed valerian oil is compounded in perfume compositions to obtain stress relieving effects more effectively, there is less problems associated with the quality of the scent of the perfume.

Subsequently, a test using the obtained reformed valerian oil (stress relieving perfume) was conducted to confirm an effectiveness of stress relieving effect.

### Test Example 2: Test for confirming the stress relieving effect

18 examinees, female university students, were given stresses by memorizing about 200 words of sentences of the Pharmaceutical Affairs Law in ten minutes and repeating it from memory in the presence of the others after a five-minute rest. After taking a rest, stress relieving perfume was sprayed from the air conditioner. To compare the differences, a similar experiment without spraying the perfume was done.

As a stress indicator, the concentration of cortisol in saliva was measured by the well-known RIA solid phase method. Concentration of cortisol in saliva was measured before giving stresses, immediately after giving stresses, and after 20 minutes from giving the stresses.

The result is shown in Fig. 1 relative to the concentration of cortisol prior to giving stress (defined as 100 %).

From Fig. 1, in the group without a spray of perfume, it is shown that the concentration of cortisol in saliva rises immediately after giving stress and after 20 minutes from giving stress more than before giving stress. On the contrary, the group in which reformed valerian oil was sprayed, showed that the concentration of cortisol in saliva declines immediately after giving stress and after 20 minutes from giving stress more than before giving stress. Accordingly, the stress relieving perfume of the invention is shown to have physiologically manifested stress relieving effects.

Moreover the individuals tested were asked if the feeling of strain continued, subjectively, while taking a test to determine the stress relieving effect of the invention. The criterion for evaluation is as follows. The result is shown in Fig. 2.

### <Criterion for evaluation>

ⓞ more than 16 out of 18 people answered they had kept the feeling of strain
○ 11 to 15 out of 18 people answered they had kept the feeling of strain
Δ 5 to 10 out of 18 people answered they had kept the feeling of strain
× less than 4 out of 18 people answered they had kept the feeling of strain

**Table 2**

| | |
|---|---|
| Durability of the feeling of strain | |
| Sprayed a reformed valerian oil | ⓞ |
| Not sprayed a reformed valerian oil | ⓞ |

From the result of Table 2, it is shown that the feeling of strain lasts though the stress relieving perfume of the invention has a stress relieving effect on physiologically manifested stress. Accordingly, the stress relieving perfume of the invention does not decline work efficiency, because it does not reduce the attention required to work productively.

In the following, examples of the stress relieving perfumes and stress relieving perfume compositions of the invention are shown. These stress relieving perfumes (Examples 2 and 3) and stress relieving perfume compositions (Examples 4 to 8) exhibit stress relieving effects when inhaled.

### Example 2: Stress relieving perfumes for perfumery

| No. | Perfume | Compounded amount (wt%) |
|---|---|---|
| 1 | Phenylethyl Alcohol | 20.0 |
| 2 | α-iso methyl ionone | 15.0 |
| 3 | Iso-E Super (*1) | 15.0 |
| 4 | Hedione (*2) | 10.0 |
| 5 | Lilial(*3) | 10.0 |
| 6 | Lyral(*4) | 8.0 |
| 7 | Hexyl Cinnamic Aldehyde | 5.0 |
| 8 | Geraniol | 3.0 |
| 9 | Eugenol | 3.0 |
| 10 | Habanolide (*5) | 3.0 |
| 11 | Bergamot oil | 2.0 |
| 12 | Muscone | 1.0 |
| 13 | Jasmine oil | 0.5 |
| 14 | Vanillin | 0.5 |
| 15 | Rose oil | 0.3 |
| 16 | Tonkabeans absolute | 0.3 |
| 17 | Benzoin resinoid | 0.3 |
| 18 | Oakmoss Absolute | 0.3 |
| 19 | Helional (*6) | 0.2 |
| 20 | Patchouli oil | 0.2 |
| 21 | Ambrette sead oil | 0.2 |
| 22 | Orris concrete | 0.1 |
| 23 | Collected fluid of aromatic trees' fragrance (*7) | 0.1 |
| 24 | Reformed valerian oil (Example 1) | 2.0 (2 wt%) |
| Total (wt%) | | 100.0 |

| | | |
|---|---|---|
| *1: Trade name of IFF Inc. | | |
| *2: Trade name of Firmenich Inc. | | |
| *3: Trade name of Givaudan Roure Inc. | | |
| *4: Trade name of IFF Inc. | | |
| *5: Trade name of Firmenich Inc. | | |
| *6: Trade name of IFF Inc. | | |
| *7: Agalloch | | |

### Example 3: Stress relieving perfumes for skin care

| Perfume | Compounded amount (wt%) |
|---|---|
| Limonene | 5 |
| Lemon oil | 5 |
| Bergamot oil | 5 |
| Cis-3-Hexenol | 0.1 |
| Geraniol | 5 |
| Phenylethyl Alcohol | 5 |
| Citronellol | 10 |
| Methyl Dihydrojasmonate | 30 |
| Jasmin oil | 3 |
| Lilial(*1) | 7 |
| Lyral(*2) | 5 |
| α-iso Methyl ionone | 7 |
| Iso-E Super(*3) | 3 |
| Cyclopentadecanolide | 3 |
| Galaxolide(*4) | 2 |
| Reformed valerian oil (Example 1) | 3 (3 wt%) |
| Total | 100 |

| | |
|---|---|
| *1: Trade name of Givaudan Roure | |
| *2: Trade name of IFF Inc. | |
| *3: Trade name of IFF Inc. | |
| *4: Trade name of IFF Inc. | |

Stress relieving perfume of above-mentioned Example 3 is used in cleansing oils, cleansing foams, cleansing creams for makeup, massage creams, massage oils, body creams, face creams, face lotions, beauty lotions and mask products.

### Example 4: Bath agent

| | |
|---|---|
| Sodium Bicarbonate | 70 wt% |
| Sodium Sulfate Anhydride | 28.8 |
| Stress reducing perfume of Example 3 | 1 |
| Coloring matter Y-202-1 | 0.2 |

Bath agent was obtained by stirring the compounds except perfume with V type mixer until the mixture was made uniform, and then adding stress relieving perfume into the mixture and stirring the mixture sufficiently until it was further made uniform.

### Example 5: Gel Aromatic

| | |
|---|---|
| Carrageenan | 3.0 wt% |
| Propylene Glycol | 2.0 |
| Propylparaben | 0.3 |
| Stress relieving perfume of Example 2 | 5.0 |
| Water | 89.7 |

Water was added into the mixture of Carrageenan, Propylene Glycol and Propylparaben while the mixture being mixed with stirring, and then the mixture was heated to 80°C while being stirred calmly. After that, the mixture was cooled to 65°C, and stress relieving perfume was added into the mixture while the mixture being stirred with homogenizor at 3000rpm to obtain uniform phase. Then aromatics were obtained finally with the resulting mixture poured into a prepared container and naturally cooled.

### Example 6: Liquid Aromatics

| | |
|---|---|
| 95 % ethanol | 25.0 wt% |
| Surface-active agent | 5.0 |
| Stress relieving perfume of Example 2 | 3.0 |
| Water | 67.0 |

An aromatic was obtained by mixing the compounds without water, and then by adding water into the mixture while the mixture stirred slowly to make the resulting mixture uniform. As a surface-active agent, Polyoxyethylene Nonylphenylether EO-13 was used.

### Example 7: Liquid Deodorizing Agents

| | |
|---|---|
| Deodouzing undiluted solution FS-500M (Shiraimatsu Pharmaceutical Co., Ltd.) | 5.0 wt% |
| 95 % ethanol | 19.0 |
| Surface-active agent | 10.0 |
| Reformed valerian oil of Example 1 | 1.0 |
| Water | 65.0 |

Deodorant (liquid type) was obtained by mixing the compounds without water, and then adding water into the mixture while the mixture stirred slowly. As a surface-active agent, Polyoxyethylene Nonylphenylether EO-10 was used.

### Example 8: Aerosol

| | |
|---|---|
| Deodorizing undiluted solution FS-500M | 5.0 wt% |
| 95 % ethanol | 29.0 |
| Reformed valerian oil of Example 1 | 1.0 |
| Water | 40.0 |
| Liquefied petroleum gas (4.0 kg/cm² 20°C) | 25.0 |

The compounds were mixed without the liquefied petroleum gas and were stirred to make the mixture uniform. Next, after putting the fixed amount of the mixture into an aerosol container and installing a valve to the container, the liquefied petroleum gas was poured into the container to obtain deodorant (aerosol type).

### Example 9: Stress relieving perfumes

| Perfume | Compounded amount (wt%) |
|---|---|
| Limonene | 5 |
| Lemon oil | 5 |
| Hexyl salicylate | 1 |
| Green base | 5 |
| Lavandin oil | 1 |
| Clary sage oil | 1 |
| Chamomile oil | 1 |
| Rosemary oil | 1 |
| Linalool | 5 |
| Linalyl acetate | 1 |
| Citronellol | 5 |
| Geranium oil | 1 |
| Rose absorute oil | 1 |
| Lyral | 5 |
| Terpineol | 1 |
| Benzyl acetate | 1 |
| Hedione | 10 |
| Hexyl cinnamic aldehyde | 5 |
| Jasmin absorute oil | 1 |
| Apple base | 5 |
| Borneol | 1 |
| Ryunou natural | 1 |
| Vertofix | 5 |
| Hinoki oil | 1 |
| Sandalore | 1 |
| Sandal wood oil | 1 |
| Pentalide | 10 |
| Ethylene brassylate | 10 |
| Valerian | 1 |
| Diproplylene glycol | 8 |
| Total | 100 |

The stress relieving perfume of above-mentioned Example 9 is used for cleansing oils, cleansing foams, cleansing creams for make-up, massage creams, massage oils, body creams, face creams, face lotions, beauty lotions and mask products.

### Example 10: Perfume for bath agents

| Bergamot oil | 15.0 wt% |
|---|---|
| Allyl amyl glycolate | 1.0 |
| Triplal | 2.0 |
| Galbanum base | 1.0 |
| Ligantraal | 1.0 |
| Cis-3-hexenol | 0.5 |
| Cis-3-hexenylace | 0.3 |
| Green tea base | 20.0 |
| Lavender oil | 3.0 |
| Geranium base | 3.0 |
| Armoise oil | 3.0 |
| Hedione | 20.0 |
| Ionone beta | 2.5 |
| hexyl cinnamic aldehyde | 7.0 |
| methyl anthranilate | 1.0 |
| Yara yara | 1.5 |
| Isobornyl acetate | 5.0 |
| Reformed valerian | 1.5 |
| Dipropylene glycol | 11.7 |
| Total | 100.0 |

Perfumes for bath agents according to this example can be used together with general ingredients of bath agents, just like the bath agents of the said Example 4.

As described above, stress relieving perfume of the invention can show its physiologically manifested stress relieving effects without decreasing work efficiency, because the perfume includes valerian oil as an active component. Also the stress relieving perfume compositions which have stress relieving effects on psychological manifestations of stress can be provided by compounding these stress relieving perfumes.

## Claims

1. A stress relieving perfume wherein the active component is valerian oil.

2. The stress relieving perfume according to claim 1, wherein fatty acids have been removed from valerian oil.

3. The stress relieving perfume according to claim 1, wherein the perfume comprises more than 0.2 % by weight of valerian oil per the perfume.

4. The stress relieving perfume composition comprising the perfume according to any one of claims 1 to 3.

5. A method of relieving stresses **characterized by** inhalation of an effective quantity of valerian oil.

6. The method according to claim 5, wherein more than 0.2 % by weight of valerian oils are contained in a perfume.

7. Use of valerian oil as a stress relieving agent.
